(19) Europäisches Patentamt
European Patent Office
Office européen des brevets

(11) EP 1 099 474 A1

(12) **EUROPEAN PATENT APPLICATION**
published in accordance with Art. 158(3) EPC

(43) Date of publication:
**16.05.2001 Bulletin 2001/20**

(21) Application number: **98938942.4**

(22) Date of filing: **21.08.1998**

(51) Int. Cl.$^7$: **B01J 20/26**, A61F 5/44, A61F 13/15

(86) International application number:
**PCT/JP98/03725**

(87) International publication number:
**WO 00/01479 (13.01.2000 Gazette 2000/02)**

(84) Designated Contracting States:
**DE GB**

(30) Priority: **03.07.1998 JP 20428098**
**05.08.1998 JP 23495798**

(71) Applicant:
**Sanyo Chemical Industries, Ltd.**
**Kyoto-shi Kyoto-fu, 605-0995 (JP)**

(72) Inventors:
- **FUJITA, M.,**
  **Sanyo Chem. Ind., Ltd.**
  **Kyoto-shi Kyoto 605-0995 (JP)**
- **TUBOTA, K.,**
  **Sanyo Chem. Ind., Ltd.**
  **Kyoto-shi, Kyoto 605-0995 (JP)**
- **TANAKA, K.,**
  **Sanyo Chem. Ind., Ltd.**
  **Kyoto-shi Kyoto 605-0995 (JP)**
- **AZEKAWA, K.,**
  **Sanyo Chem. Ind., Ltd.**
  **Kyoto-shi, Kyoto 605-0995 (JP)**

(74) Representative:
**VOSSIUS & PARTNER**
**Siebertstrasse 4**
**81675 München (DE)**

# (54) DEODORANT AND ANTIMICROBIAL WATER ABSORBENT, PROCESS FOR THE PRODUCTION THEREOF AND ABSORBENT ARTICLES

(57) A deodorant and antibacterial water-absorbing agent comprising a water-absorbent resin (A), a compound (B) having an antibacterial function with respect to ammonia-producing bacteria, and an agent (C) having a neutralization ability or neutralization and adsorption abilities with respect to ammonia, wherein (A) and (B) are mixed and/or bonded; processes for producing the water-absorbing agent; and absorbent articles having an absorption layer in which the water-absorbing agent is held by a support comprising a fibrous material. The water-absorbing agent of the present invention is excellent in deodorant and antibacterial functions as well as in absorption capacities, which is essential for a water-absorbing agent, and powder handling properties (powder flowability, hygroscopic blocking resistance). Absorbent articles using this water-absorbing agent absorb liquids such as urine, blood, body fluids and the like, and display deodorant and antibacterial effects. The absorbent articles are useful in applications such as disposable diapers for adults and infants, incontinence pads, sanitary napkins, panty liners, mother's milk pads, parturition mats, underpads for medical treatment, and the like.

EP 1 099 474 A1

## Description

TECHNICAL FIELD

The present invention relates to a water-absorbing agent, a process for producing the same, and absorbent articles. Particularly, the present invention relates to a water-absorbing agent having deodorant and antibacterial properties, which comprises a water-absorbent resin, a compound having an antibacterial function with respect to ammonia-producing bacteria and an agent having a neutralization ability or neutralization and adsorption abilities with respect to ammonia; a process for producing the water-absorbing agent; and absorbent articles using the water-absorbing agent, which absorb liquids that generate offensive odors by putrefaction, particularly urine, blood, body fluids and the like.

BACKGROUND ART

Water-absorbent resins are used in various applications, for example, absorbent articles such as disposable diapers, incontinence pads, sanitary napkins, mother's milk pads and the like, pet sheets, animal litter, excretion treating agents, waste blood gelling agents, drip absorbing agents, freshness retaining agents, etc., utilizing its absorption capacities, water-holding capacity and gelling ability. Although conventional water-absorbent resins are excellent in the abilities of absorbing and holding liquids such as urine, blood, body fluids and the like, they do not have an antibacterial function and have little deodorant effect.

Thus, when a conventional water-absorbent resin is caused to absorb a liquid such as urine, blood, body fluids or the like, there is a problem that organic substances contained in the absorbed liquid are decomposed by fungi, microorganisms and the like or by air oxidation, and the decomposed products cause generation of an offensive odor, irritation on the skin, rash, and so on. In addition, the liquid-absorbed gel is putrefied easily by fungi and microorganisms contained in the absorbed liquid or by bacteria or the like in the air, and may emit an offensive odor by putrefaction. Furthermore, an offensive odor is generated by ammonia formed by decomposition of urea in body fluids by enzymes such as urease, etc. Thus, in view of the environmental and sanitary aspects and safety, it has been desired to realize materials satisfying both absorption function and deodorant and antibacterial functions. Particularly, the problems of offensive odors and rash are serious in absorbent articles such as disposable diapers for adults, which are used for bedridden elderly people or patients, etc., and it has been desired to solve the problems.

Traditionally, to improve an antibacterial or deodorant property of water-absorbent resins, the following compositions (i) and (ii), etc. have been proposed:

(i) water-absorbing agent compositions in which an organic acid salt of a specific amine compound or a quaternary ammonium salt is contained or supported in a water-absorbent resin (JP 9(1997)-248454 A); and

(ii) water-absorbing agent compositions in which a porous inorganic powder that has absorbed an antibacterial component is mixed with a water-absorbent resin (JP 10 (1998)-120921 A). It also has been proposed to use these compositions in absorbent articles.

Although these compositions are excellent in an antibacterial action and can prevent generation of an offensive odor resulting from putrefaction, they have little effect to inhibit an offensive odor not resulting from putrefaction (an ammonia odor generated by decomposing urea in a body fluid), and are not satisfactory with respect to a deodorant property. Thus, when these compositions are used in absorbent articles, satisfactory effects to prevent generating an offensive odor and to inhibit putrefaction have not necessarily been obtained.

SUMMARY OF THE INVENTION

It is an object of the present invention to provide a water-absorbing agent that is excellent in deodorant and antibacterial functions as well as in absorption capacities, which is essential for a water-absorbing agent, and powder handling properties (powder flowability, hygroscopic blocking resistance), and a process for producing the same; and further to provide absorbent articles using the water-absorbing agent, particularly that can absorb liquids such as urine, blood, body fluids and the like that generate offensive odors by putrefaction and can display deodorant and antibacterial effects. The absorbent articles are useful in various applications such as disposable diapers for adults and infants, incontinence pads, sanitary napkins, panty liners, mother's milk pads, parturition mats, underpads for medical treatment, and the like.

That is, the present invention relates to a water-absorbing agent [1]; processes [2] and [3] for producing the water-absorbing agent; and absorbent articles using the water-absorbing agent [1] as follows:

[1] Water-absorbing agent

A deodorant and antibacterial water-absorbing agent comprising: a water-absorbent resin (A); a compound (B) having an antibacterial function with respect to ammonia-producing bacteria; and an agent (C) having a neutralization ability or neutralization and adsorption abilities with respect to ammonia, wherein (A) and (B) are mixed and/or bonded.

[2] Process for producing a water-absorbing agent

A process for producing a deodorant and antibacterial water-absorbing agent, comprising: mixing and kneading (B) with a hydrous gel of (A); drying and pulverizing the mixture; and then mixing (C) therewith.

[3] Process for producing a water-absorbing agent

A process for producing a deodorant and antibacterial water-absorbing agent, comprising: mixing a powder of (A) and an aqueous solution or aqueous dispersion of (B); and then mixing an aqueous solution, aqueous dispersion or fine powder of (C) with the mixture, thereby adhering (C) to the surface of (A).

[4] Absorbent article

An absorbent article having an absorption layer in which a deodorant and antibacterial water-absorbing agent obtainable by the process [2] or [3] is held by a support comprising a fibrous material.

DETAILED DISCLOSURE OF THE INVENTION

*Specific Examples of Water-absorbent Resin (A)*

As the water-absorbent resin (A), because (A) is expected to have a certain degree of function of adsorbing ammonia, it is preferable to use a water-absorbent resin that is water-insoluble and has a carboxylic acid group as a constituent unit, and the kind and production process thereof are not particularly limited.
Examples of the water-absorbent resin (A) that may be used suitably in the present invention are as follows:

crosslinked copolymers of starch-acrylic acid (acrylate), described in JP 53 (1978)-46199 B and JP 53 (1978)-46200 B, etc.;
crosslinked or self-crosslinked polyacrylates by reverse phase suspension polymerization, described in JP 54 (1979)-30710 B and JP 56 (1981)-26909 A, etc.;
crosslinked polyacrylic acids (or salts thereof) obtained by aqueous solution polymerizations (adiabatic polymerization, thin film polymerization, spray polymerization, etc.), described in JP 55 (1980)-133413 A, etc.;
saponified copolymers of vinyl esters with unsaturated carboxylic acids or derivatives thereof, described in JP 52 (1977)-14689 A and JP 52 (1977)-27455 A, etc.;
crosslinked polyacrylic acids (or salts thereof) copolymerized with sulfonic acid (or salts thereof) group-containing monomers, described in JP 58 (1983)-2312 A and JP 61 (1986)-36309 A, etc.;
crosslinked isobutylene-maleic anhydride copolymers;
hydrolyzed starch-acrylonitrile copolymers;
crosslinked carboxymethyl cellulose derivatives; and
crosslinked copolymers of acrylic acid (or salts thereof) and acrylamide.

Combinations of two or more kinds of the above water-absorbent resins also may be used.
Among these preferred are water-insoluble water-absorbent resins having acrylic acid and salts thereof (acrylate) as main constituent units, since they display relatively large absorption capacities.
As the water-insoluble water-absorbent resins having acrylic acid and salts thereof as main constituent units, it is preferable that the acrylic acid component is at least 20 to 50 mol % with respect to the total of the acrylic acid component and the acrylate component. More preferably, the mole ratio of the acrylic acid component is 25 to 40 mol %. Although the salt of the acrylate is usually a sodium salt and/or a potassium salt, it may be an organic salt such as ammonium salt, amine salt or the like depending on the use.
Thus, by leaving acrylic acid units in the water-absorbent resin, an accompanying effect is obtained that the carboxyl groups in the acrylic acid component adsorb ammonia or the like, which is a kind of odor component. By making the mole ratio of acrylic acid units with respect to the total of the acrylic acid component and the acrylate component

in the range of not more than 50 %, the obtained deodorant and antibacterial water-absorbing agent has excellent absorption capacities, and exhibits a neutral or weakly acidic pH, which is preferable in respect of safety to the skin. On the other hand, by making the mole ratio of the acrylic acid units in the range of at least 20 %, the obtained deodorant and antibacterial water-absorbing agent has an excellent effect of adsorbing ammonia or the like, and exhibits a neutral or weakly acidic pH, which is also preferable with respect to safety to the skin.

Furthermore, a surface crosslinked water-absorbent resin in which powder of the above-described water-absorbent resin is further crosslinked in the vicinity of the surface also may be used suitably in the present invention. Examples of the crosslinking agent used in the surface crosslinking include polyglycidyl compounds having 2 to 10 epoxy groups in a molecule such as ethylene glycol diglycidyl ether, glycerol diglycidyl ether, polyglycerol polyglycidyl ether, etc.; polyhydric alcohols having 2 to 20 carbon atoms such as glycerol, ethylene glycol, etc.; alkylene carbonates having 2 to 10 carbon atoms in an alkylene group; polyamine resins (molecular weight of 200 to 500,000) such as polyamide-polyamine-epichlorohydrin resin, polyamine-epichlorohydrin resin, etc. Polyglycidyl compounds and polyamine resins are preferable in that a crosslinking reaction can be carried out at a relatively low temperature. These crosslinking agents may be used either alone or in combinations of two or more kinds.

In the absorption capacities of the obtained water-absorbent resin (A) with respect to physiological saline solution (0.9 mass % sodium chloride aqueous solution), the absorbency under pressure-free state is usually at least 30 g/g, preferably 35 to 80 g/g, more preferably 40 to 75 g/g. The absorption capacities are measured according to the procedures described below.

The shape of the water-absorbent resin (A) is not particularly limited as long as it is powdery. For examples, the shape may be any of particle, granule, pellet, flake, lump, sphere, fine particle, and the like. The particle size or particle size distribution also is not particularly limited, however, the particle size of at least 90 mass % is usually not more than 1 mm, preferably 0.1 to 0.9 mm.

*Specific Examples of Compounds (B) Having Antibacterial Function with Respect to Ammonia -producing Bacteria*

The compound (B) of the present invention having an antibacterial function with respect to ammonia-producing bacteria is not particularly limited, as long as it has an antibacterial function with respect to ammonia-producing bacteria. Examples of (B) include a quaternary ammonium salt compound (B1) having at least one $C_6$ to $C_{30}$ alkyl group, a polymethylene biguanidine compound (B2), a chlorohexidine compound (B3), and the like. The quaternary ammonium salt compound (B1) having at least one $C_6$ to $C_{30}$ alkyl group is preferable.

Examples of the alkyl group in (B1) include hexyl, octyl, decyl, lauryl, myristyl, cetyl, oleyl, stearyl, and behenyl groups. When the alkyl group has 6 to 30 carbon atoms, an excellent antibacterial property is displayed. Although residues other than $C_6$ to $C_{30}$ alkyl groups in the quaternary ammonium compounds are not particularly limited, examples thereof include $C_1$ to $C_5$ alkyl groups, hydroxyalkyl groups such as 2-hydroxyethyl group, araliphatic groups such as benzyl group, aromatic groups such as phenyl group, and the like. Although the number of quaternary ammonium groups in a molecule is not particularly limited, it is preferable that (B1) has one quaternary ammonium group in a molecule.

Specific examples of (B1) are compounds having the quaternary ammonium groups listed below as cation components and having the organic or inorganic acid anions listed below as anion components. Preferred are quaternary ammonium salt compounds having organic acid anions as anion components.

(1) Quaternary ammonium groups as a cation component in the quaternary ammonium salt compound (B1):

Examples of the quaternary ammonium groups include hexyltrimethylammonium, octyltrimethylammonium, decyltrimethylammonium, lauryltrimethylammonium, cetyltrimethylammonium, stearyltrimethylammonium, octyldimethylethylammonium, decyldimethylethylammonium, lauryldimethylethylammonium, dihexyldimethylammonium, dioctyldimethylammonium, didecyldimethylammonium, didodecyldimethylammonium, dilauryldimethylammonium, and the like.

Among these, preferred are quaternary ammonium groups having at least one $C_8$ to $C_{20}$ alkyl group, more preferably quaternary ammonium groups having at least one $C_8$ to $C_{12}$ alkyl group, particularly preferably quaternary ammonium groups having two $C_8$ to $C_{12}$ alkyl groups in a molecule (dioctyldimethylammonium, didecyldimethylammonium, etc.).

(2) Organic acids as an anion component in the quaternary ammonium salt compound (B1):

Examples of the organic acids include carboxylic acids, sulfonic acids, organic phosphoric acids, and the like.

(i) Carboxylic acids:

saturated monocarboxylic acids having 1 to 30 carbon atoms (acetic acid, propionic acid, capric acid, lauric acid, myristic acid, stearic acid, behenic acid, etc.), unsaturated monocarboxylic acids (acrylic acid, methacrylic acid, oleic acid, etc.), aliphatic oxycarboxylic acids (glycolic acid, lactic acid, gluconic acid, etc.), aliphatic polycarboxylic acids (oxalic acid, succinic acid, adipic acid, azelaic acid, sebacic acid, maleic acid, fumaric acid, itaconic acid, etc.), and aromatic carboxylic acids (phthalic acid, trimellitic acid, pyromellitic acid, etc.), and the like.

(ii) Sulfonic acids:

aliphatic sulfonic acids having 1 to 30 carbon atoms (methanesulfonic acid, ethanesulfonic acid, laurylsulfonic acid, etc.), aromatic sulfonic acids (p -toluenesulfonic acid, naphthalenesulfonic acid, etc.), and the like.

(iii) Organic phosphoric acids:

aliphatic alkyl phosphoric mono- or diesters having 1 to 30 carbon atoms, aliphatic alkyl phosphinic acids having 1 to 30 carbon atoms, aliphatic alkyl phosphonic acids having 1 to 30 carbon atoms, and the like.

(3) Inorganic acids as an anion component in the quaternary ammonium salt compound (B1):

Examples of the inorganic acids include hydrofluoric acid, hydrochloric acid, bromic acid, iodic acid, sulfuric acid, nitric acid, phosphoric acid, carbonic acid, boric acid, and the like.

Organic acids are preferable. Among the organic acids, preferred are carboxylic acids, more preferably monocarboxylic acids, aliphatic oxycarboxylic acids, aliphatic polycarboxylic acids, particularly preferably gluconic acid and adipic acid. Among the inorganic acids, preferred are hydrochloric acid, sulfuric acid, phosphoric acid, carbonic acid, and boric acid.

When the anion component of a quaternary ammonium group-containing compound is an inorganic acid anion, by using the inorganic salt of an acid selected from strong acids and carbonic acid described below in combination with powder of the water-absorbing agent, both deodorant and antibacterial properties are displayed excellently, even though the anion component of the quaternary ammonium salt compound is an inorganic acid.

Examples of the polymethylene biguanidine compound (B2) include polyhexamethylene guanidine salt compounds, polyoctamethylene guanidine salt compounds, and the like, and the pairing anion thereof may be either of an organic acid or inorganic acid. As the organic acid or inorganic acid, those listed in (2) and (3) for (B1) may be used.

Examples of the chlorohexidine compound (B3) include chlorohexidine and organic acid salts (chlorohexidine gluconate, etc.) and inorganic acid salts (chlorohexidine hydrochloride, etc.) thereof, and the like.

*Specific Examples of Forms in Which a Water-absorbent Resin and a Compound Having an Antibacterial Function with Respect to Ammonia-producing Bacteria are Bonded.*

Although (A) and (B) usually exist in a mixed form, the water-absorbing agent of the present invention displays the same effect in a bonded form. When the water-absorbent resin (A) and (B1) exist in a bonded form, it is preferable that (B) is the quaternary ammonium salt compound (B1) having at least one $C_6$ to $C_{30}$ alkyl group. In this case, (B1) exists in (A) in the form of a quaternary ammonium salt, and may be a group shown by the following general formula (1):

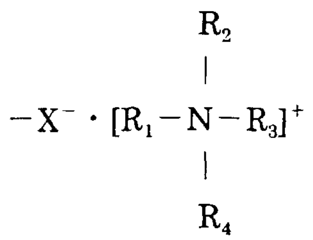

(1)

where $X^-$ is an anion radical covalently bonded to a polymer chain of (A), and the quaternary ammonium groups

in residues (including $R_1$ to $R_4$) correspond to the above-described quaternary ammonium groups of (1) in (B1).

That is, it has such a structure that at least one acid radical covalently bonded to a polymer chain of (A) becomes an anion radical $X^-$with respect to a quaternary ammonium cation to form a salt.

The $X^-$ is not particularly limited, and may be any anion radical covalently bonded to a polymer chain. For example, $X^-$ may be any of carboxyl anion radical $-COO^-$, sulfonic acid anion radical $-SO_3^-$, sulfuric acid anion radical, phosphoric acid anion radical, and the like, preferably carboxyl anion radical or sulfonic acid anion radical, particularly preferably carboxyl anion radical.

The content of quaternary nitrogen atoms in (A) is usually in the range of $2 \times 10^{-4}$ to 0.8 mass %, preferably $1 \times 10^{-3}$ to 0.4 mass %, particularly preferably $2 \times 10^{-3}$ to 0.1 mass % with respect to the mass of (A), and at least a part of the acid radicals exist in the form of a quaternary ammonium salt. The remaining acid radicals in (A) that are not in the form of a quaternary ammonium salt are usually non-neutralized acid radicals, and acid radicals neutralized to form alkali metal salts, ammonium salts or amine salts.

The form in which (A) and (B1) are bonded usually is obtained by the reaction between acid radicals in (A) and quaternary ammonium carbonate in (B1). In this reaction, a carbonate anion in (B1) and at least one of acid radicals in (A) carry out anion exchange. That is, because an anion in (A) reacts to form an anion component with respect to a quaternary ammonium cation in (B1), and the carbonate anion reacts to form carbonate gas and is released, there is the advantage that no by-products remain, and a quaternary ammonium salt group-containing water-absorbent resin of high purity can be obtained. It does not mean that the above reaction necessarily occurs by 100 %, but a certain degree of unreacted (B1) may remain in (A). That is, the state as a quaternary ammonium cation forming a salt and the state of a quaternary ammonium carbonate may coexist.

Examples of a quaternary ammonium carbonate (b) that can be used to be bonded with (A) include compounds shown by a general formula (2) or (3) below:

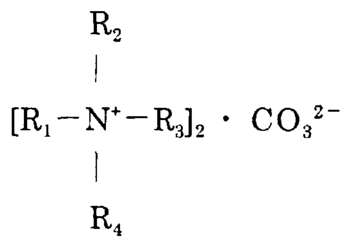

(2)

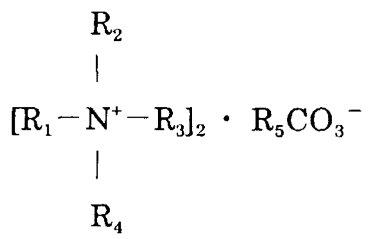

(3)

where $R_1$ to $R_4$ are selected from the same organic groups as described above for the quaternary ammonium groups of (B1), and $R_5$ is a lower alkyl group having 1 to 6 carbon atoms.

When (B1) is a compound of the general formula (2), although it forms lower alcohols as well as carbonate gas by the reaction with (A), because these are dissolved in water or vaporized, a resin (A + B1) of high purity also can be obtained without leaving by-products.

The compounds shown by the general formula (2) or (3) can be synthesized, for example, by the reaction between a tertiary amine (b1-1) having at least one $C_6$ to $C_{30}$ alkyl group and a carbonic diester (b1-2).

Examples of (b1-1) include octyldimethylamine, decyldimethylamine, lauryldimethylarnine, myristyldimethylamine, cetyldimethylamine, stearyldimethylamine, dihexylmethylamine, dioctylmethylamine, didecylmethylamine, didodecylmethylamine, 2-beptadecenyl-hydroxyethylimidazoline, and the like. Tertiary amines having two $C_6$ to $C_{20}$ aliphatic alkyl groups are preferable, more preferably dioctylmethylamine and didecylmethylamine.

Examples of (b1-2) include dimethyl carbonate, diethyl carbonate, ethyl methyl carbonate, dipropyl carbonate, and the like. Dimethyl carbonate having a small number of carbon atoms in an alkyl group is preferable because of

its excellent reactivity.

When producing the quaternary ammonium carbonate, the mole ratio between the tertiary amine (b1-1) and the carbonic diester (b1-2) is usually 1 : (0.3 to 4.0), preferably 1 : (0.5 to 2.5). The reaction temperature is usually 30 to 150 °C, preferably 50 to 120 °C. Solvents such as methanol and ethanol, or these solvent mixed with water may be used as needed.

When producing (A + B1), the ratio of (A) to (B1) may be any ratio such that the content of quaternary nitrogen atoms in (A) is in the above-mentioned range, and may vary depending on the intended balance between the absorption capacities and the deodorant and antibacterial functions.

The ratio of (A) : (B1) for the equivalent ratio of acid radicals in (A) to (B1) is usually 1 : ($3 \times 10^{-5}$ to 0.3), preferably 1 : ($1 \times 10^{-4}$ to 0.1), more preferably 1 : ($3 \times 10^{-4}$ to $8 \times 10^{-2}$).

When the equivalent ratio of (B1) is at least $3 \times 10^{-5}$, the obtained water-absorbent resin has excellent deodorant and antibacterial effects. On the other hand, sufficient deodorant and antibacterial effects are displayed when the equivalent ratio of (B) is 0.3. Thus, it is not necessary to increase the ratio to greater than 0.3, since the deodorant and antibacterial effects hardly increase.

More specifically, (A + B1) is obtained by reacting the water-absorbent resin (A) having an acid radical with a quaternary ammonium carbonate compound (B1) in the presence of water, for example, by the following processes (1) to (4). Among these, preferred are the processes (1) and (4).

(1) Mixing an aqueous solution of a quaternary ammonium carbonate (B1) with powder of the water-absorbent resin (A), and then reacting the mixture, thereby forming a quaternary ammonium salt in the vicinity of the surface of the powder of the water-absorbent resin.
(2) Mixing and kneading (B1) with a hydrous gel polymer obtained in the process of producing (A), then heating the mixture and allowing it to react and dry, and pulverizing the mixture.
(3) Causing (A) to absorb water to form a hydrous gel, then mixing and kneading (B1) with the hydrous gel, heating the mixture and allowing it to react and dry, and pulverizing the mixture.
(4) Causing (A) to absorb an aqueous solution of (B1) to form a hydrous gel, kneading the hydrous gel, then heating the hydrous gel and allowing it to react and dry, and pulverizing the hydrous gel.

The amount of water used in the processes (1) to (4) is usually 1 to 500 mass parts, preferably 5 to 450 mass parts with respect to 100 mass parts of (A). When the amount of water is not less than 1 mass part, the reaction between (A) and (B1) proceeds sufficiently. On the other hand, when the amount of water is more than 500 mass parts, although the reaction between (A) and (B1) is not hindered, it is required to vaporize a large amount of water after the reaction, and this is not economical.

In the above processes (1) to (4), the industrial apparatus for mixing (A) and (B1) is not particularly limited, and a conventional apparatus may be used. Examples of the industrial apparatus for mixing powder of (A) and an aqueous solution of (B1) include universal mixer, turbulizer, Nauta blender, ribbon blender, conical blender, V-shaped mixer, screw mixer, fluidized bed mixer, spray type mixer, mortar mixer, and the like. Examples of the industrial apparatus for mixing and kneading (B1) with a hydrous gel or water-absorbed gel of (A) include kneader, uniaxial/biaxial type extruding mixer, universal mixer, gear compounder, meat grinder, Nauta blender, screw mixer, and the like.

Although the temperature at which the reaction of (A) and (B1) is carried out is not particularly limited, for example, the following methods (i) and (ii) may be employed.

(i) When the water-absorbent resin (A) is a powder when reacted as in the process (1), it is preferable that the temperature is from 1 to 150 °C, particularly from 10 to 130 °C.
(ii) When the water-absorbent resin (A) is a hydrous gel when reacted, and drying is required as well as reacting as in the processes (2) to (4), it is preferable that the temperature is from 40 to 150 °C, particularly preferably from 40 to 130 °C. The reacting and drying also may be carried out under a reduced pressure as needed.

When the temperature is not lower than 1 °C, freezing of water does not occur, so that liquid homogeneity is maintained. When the temperature is not higher than 150 °C, (B1) is not decomposed due to heat, so that it is preferable with respect to reaction.

Furthermore, a crosslinking agent having at least two functional groups that can react with acid radicals in (A) also may be used so that a crosslinked structure is introduced simultaneously with the reaction of (A) and (B1). As this crosslinking agent, the same crosslinking agents as used when producing the water-absorbent resin described above may be employed.

The water-absorbing agent of the present invention may contain a general water-absorbent resin (A') not having a quaternary ammonium group as well as (A + B1). But it is preferable that both are mixed homogeneously so that deodorant and antibacterial properties are displayed homogeneously.

*Specific Examples of the Agent (C) Having a Neutralization Ability or Neutralization and Adsorption Abilities with Respect to Ammonia*

In the present invention, the agent (C) having a neutralization ability or neutralization and adsorption abilities with respect to ammonia is not particularly limited, as long as it has a function of neutralizing ammonia or functions of neutralizing and adsorbing ammonia. For example, an inorganic acid salt (C1), a flavonoid compound (C2), a cyclodextrin (C3), a chelate compound, a metal complex compound, or the like may be used as (C). Among these, preferred is an inorganic acid salt (C1).

Examples of the inorganic acid salt (C1) include inorganic salts of an acid selected from strong acids and carbonic acid. Specifically, for example, a salt of an inorganic strong acid (hydrochloric acid, bromic acid, sulfuric acid, nitric acid, boric acid, phosphoric acid, etc.) and a weak base (ammonia, aluminum hydrate, etc.) and/or an inorganic hydrogencarbonate, etc. may be used. Specific examples thereof are sulfates such as aluminum sulfate, aluminum ammonium sulfate, aluminum potassium sulfate and ferric ammonium sulfate; hydrochlorides such as ammonium chloride and aluminum polychloride; nitrates such as ammonium nitrate; borates such as sodium tetraborate and sodium metaborate; phosphates such as aluminum dihydrogen tripolyphophate; hydrogencarbonates such as sodium hydrogencarbonate and ammonium hydrogencarbonate; and hydrates of these compounds. Mixtures of two or more kinds of these compounds also may be used.

Particularly, alums are mixtures of sulfates of various metals (double salts), and examples thereof include aluminum iron alum, potassium aluminum alum (also called potassium alum), ammonia alum, sodium alum, and the like.

Preferred as (C1) are alum compounds, particularly preferably sodium alum (hydrate of aluminum sodium sulfate) and potassium alum (hydrate of aluminum potassium sulfate).

The pH of 1 mass % aqueous liquid of (C1) is usually 3.0 to 6.5, preferably 3.5 to 6.0, more preferably 4.0 to 5.5. When the pH of (C) is not less than 3.0, it causes no irritation on the skin when contacting the skin, and this is preferable. Furthermore, when the pH of (C) is not more than 6.5, the deodorant effect is excellent, and this is preferable. The aqueous liquid herein refers to an aqueous solution or an aqueous dispersion.

It is not always necessary that the flavonoid compound (C2) is a single compound, and (C2) is not particularly limited as long as it is an agent containing a flavonoid compound. For example, extracts of Theaceae plants or tea-leaf, extract of grapefruit seed, and the like contain a flavonoid compound or flavonoid component, and are used suitably in the present invention. Examples of (C2) on the market are "FRESHSHIRAIMATSU" (manufactured by SHIRAIMATSU PHARMACEUTICAL CO., LTD.), "SMELL-LULL" (manufactured by KANKYO KAGAKU KAIHATSU Corporation) "ZEONCLEAN" (manufactured by ZEON CHEMICAL Corporation), "AMOLDEN" (manufactured by DAIWAKAGAKU KOGYO Corporation), and the like.

Examples of the cyclodextrin (C3) include α-cyclodextrin, β-cyclodextrin, γ-cyclodextrin, derivatives thereof [those obtained by methylation, ethylation, hydroxyethylation, hydroxypropylation, maltose bonding, cationization (quaternization, etc.), anionization, amphoterization, and the like], and mixtures thereof.

*Ratio of (A), (B) and (C)*

Although the ratio of (A), (B) and (C) mixed in the present invention can be varied depending on the intended balance of the absorption capacities and the deodorant and antibacterial functions, etc., the ratio of (A) : (B) : (C) is usually 100 : (0.01 to 5) : (0.05 to 10), preferably 100 : (0.05 to 3) : (0.1 to 7), more preferably 100 : (0.1 to 2) : (0.2 to 5) on mass basis.

When the ratio of (B) with respect to the mass of (A) is at least 0.01 mass %, the obtained water-absorbing agent exhibits an excellent antibacterial effect. On the other hand, because a sufficient antibacterial effect is displayed at a ratio of 5 mass % of (B) with respect to the mass of (A), it is preferable economically that the ratio is not more than 5 mass %. Furthermore, when the ratio of (C) with respect to the mass of (A) is at least 0.05 mass %, immediate effectiveness of the deodorant effect is excellent. On the other hand, when the ratio of (C) with respect to the mass of (A) is not more than 10 mass %, both the deodorant and antibacterial functions and the absorption capacities are excellent, and thus is preferable. By increasing the ratio of (C), the immediate effectiveness of the deodorant function can be improved. On the other hand, by increasing the ratio of (B), an offensive odor generated by the action of bacteria or ammonia-producing bacteria, etc. can be controlled to a low level over a long time, and an antibacterial effect also is improved.

In the case of a form in which (A) and (B) are bonded, the ratio of (A + B) : (C) is usually 100 : (0.05 to 10), preferably 100 : (0.1 to 7), more preferably 100 : (0.2 to 5) on mass basis.

When the ratio of (C) with respect to the mass of (A + B) is at least 0.05 mass %, immediate effectiveness of the deodorant effect is excellent. On the other hand, when the ratio of (C) with respect to the mass of (A + B) is not more than 10 mass %, both the deodorant and antibacterial functions and the absorption capacities are excellent, and

thus is preferable.

*Process for Producing Water-absorbing Agent*

In the present invention, for example, (B) and (C) are applied to (A) by the processes as follows: (i) making (B) to be built in (A) and then treating it with (C); (ii) making (B) to be supported by (A) and then treating it with (C); (iii) mixing (A), (B) and (C); and (iv) treating (A) with (C) and then making (B) to be supported by (A) treated with (C).

In the process (i), (B) is built in (A), for example, by the following processes (1) to (3).

(1) (B) is built in (A) by mixing and kneading (B) with a hydrous gel of (A) obtained by causing (A) to absorb water, and drying and pulverizing the mixture.

(2) (B) is built in (A) by mixing and kneading (B) with a hydrous gel polymer obtained in the process of producing (A), and drying and pulverizing the mixture.

(3) A part of (B) is built in (A) in the vicinity of the surface of (A) by admixing an aqueous solution or aqueous dispersion of (B) with powder of (A).

A water-absorbing agent in which (B) is built in (A) also can be obtained by dissolving or dispersing (B) in a polymerization liquid obtained in the process of producing (A), thereafter polymerizing it, and drying and pulverizing it. However, this process is not preferable because the antibacterial effect of (B) may be deactivated during the polymerization.

In the process (ii), (B) is supported by (A), for example, by the following processes (4) to (6).

(4) (B) is supported by (A) by mixing powder of (A) with (B) in advance, and then adding a small amount of water to the mixture, without drying or with drying as needed.

(5) (B) is supported by (A) by adding an aqueous liquid of (B) to powder of (A) and mixing them, without drying or with drying as needed.

(6) When (A) is a surface crosslinked water-absorbent resin, (B) is supported by (A) by mixing (B) in a surface crosslinking agent solution, or adding a surface crosslinking agent solution to (A) and thereafter admixing (B) with (A).

In the processes (i), (ii) and (iv), (A) having a built-in or supported (B), or (A) alone is treated with (C), for example, by the process as follows:

(7) Mixing (C) in the form of an aqueous liquid or fine powder.

The processes (i)-(2), (i)-(3), (ii)-(5) and (iv) are preferable in that (A), (B) and (C) exist together relatively homogeneously, and that (A) can be treated during the process of its production, or drying and pulverizing are not necessarily required.

The industrial apparatus for mixing (A) and (B) in the presence or absence of water in the processes (1) to (5), or the industrial apparatus for mixing (C) in the presence or absence of water in the process (7) is not particularly limited, and conventional apparatuses may be employed.

Examples of the industrial apparatus include a kneader, uniaxial or multiaxial type extruding mixer, universal mixer, turbulizer, Nauta blender, ribbon blender, conical blender, V-shaped mixer, screw mixer, and the like.

Particularly, because the ionic affinity between (A) having a non-neutralized carboxylic acid group and (B1) having a cation radical is high, by treating first (A) and (B1) to adhere both components, and then treating it with (C) having an anion radical, a water-absorbing agent can be obtained that has a high mixture homogeneity in which three components have excellent adherence and are hard to be separated by vibration, etc. In the thus obtained water-absorbing agent, deterioration of powder flowability, which usually is caused by treatment only with an antibacterial component, does not occur, and further hygroscopic blocking resistance (hygroscopic blocking is a characteristic that particles of a water-absorbent resin block one another at a high humidity and powder handling properties are reduced) also is improved.

In the water-absorbing agent of the present invention, organic powders (e.g. pulp powder, cellulose derivatives, natural polysaccharides, etc.), antioxidants, surfactants, coloring agents, perfumes, fine particulate silica, and the like may be blended as extenders or additives in an arbitrary step as needed. The amounts thereof are usually not more than 5 mass % with respect to the mass of the deodorant and antibacterial water-absorbing agent.

The shape and the particle size distribution of the deodorant and antibacterial water-absorbing agent of the present invention are not particularly limited. The shape may be any of particle, granule, pellet, flake, lump, sphere, fine particle, and the like. The particle size distribution also is not particularly limited, and the particle size of at least 90 mass % is usually 0.05 to 1 mm, preferably 0.1 to 0.9 mm.

*Performances of the Water-absorbing Agent*

The deodorant and antibacterial water-absorbing agent of the present invention exhibits excellent effects in both deodorant and antibacterial properties. For example, with respect to the deodorant property, in an ammonia deodorization test, the ammonia gas concentration after allowing the water-absorbing agent to stand at 37 °C for 10 minutes is preferably not more than 50 %, particularly preferably not more than 30 %, of the initial ammonia gas concentration.

With respect to the antibacterial property, in an antibacterial property test, the viable cell number measured by a mixture plane culturing method after inoculating ammonia-producing bacteria and culturing them at 37°C for 2 hours is not more than one ten-thousandth, preferably one hundred-thousandth, of the initial viable cell number at the time of inoculation (blank).

Furthermore, in the absorption capacities of the deodorant and antibacterial water-absorbing agent of the present invention with respect to an artificial urine, the absorbency under pressure-free state is usually at least 30 g/g, preferably 35 to 80 g/g, more preferably 40 to 75 g/g; the absorbency under load (60 g/cm$^2$) is at least 15 g/g, preferably at least 18 g/g, more preferably at least 20 g/g; the absorbency under load (60 g/cm$^2$) of sheared gel calculated by the formula below is at least 10 g/g, preferably at least 13 g/g, more preferably at least 15 g/g; and the retention ratio of absorbency under load (60 g/cm$^2$) of sheared gel is 60 to 100 %, preferably 70 to 100 %, more preferably 80 to 100 %. The absorption capacities are measured using an artificial urine composition described below in accordance with the procedure described below.

$$\text{Retention ratio of absorbency under load (60 g/cm}^2\text{) of sheared gel (\%)} = [\text{Absorbency under load (60 g/cm}^2\text{) after shearing / Absorbency under load (60 g/cm}^2\text{) before shearing}] \times 100$$

*Water Aborbent Article*

By using the deodorant and antibacterial water-absorbing agent of the present invention in various types of absorbent articles, articles can be obtained that satisfy sufficiently both absorption capacities and deodorant and antibacterial effects.

The deodorant and antibacterial water-absorbing agent may be used in absorbent articles by any method, as long as a water absorption layer is formed in which the deodorant and antibacterial water-absorbing agent is held by an absorbent support comprising a fibrous material (D). Examples of the method are as follows:

(1) dispersing particles of the deodorant and antibacterial water-absorbing agent between layers in which a fibrous material comprising pulp, thermal-adhesive fiber, and the like is arranged in a layer form;
(2) mixing a fibrous material comprising pulp, thermal-adhesive fiber, and the like with the deodorant and antibacterial water-absorbing agent; and
(3) sandwiching the deodorant and antibacterial water-absorbing agent and as needed a fibrous material between two or more water absorbing papers or nonwoven fabrics.

As the fibrous material (D), for example, fibrous materials traditionally used in absorbent articles such as various types of fluff pulp and cotton-like pulp may be used. Raw materials (conifer, broad-leaved tree, etc.), process of production (chemical pulp, semichemical pulp, chemithermomechanical pulp (CTMP), etc.), bleaching process, and the like are not limited particularly.

Furthermore, as the fibrous material (D), synthetic fiber not swellable in water also may be used as needed, either alone or in combination with the fluff pulp, cotton-like pulp or the like described above. Examples of the synthetic fiber include polyolefin fibers (e.g. polyethylene-based fiber, polypropylene-based fiber), polyester-based fibers (e.g. polyethylene terephthalate fiber), polyolefin/polyester conjugated fiber, polyamide-based fiber, polyacrylonitrile-based fiber, and the like.

The length and thickness of (D) are not particularly limited, but usually, it is preferable that the length is 1 to 200 mm and the thickness is 0.1 to 100 denier.

The shape of (D) also is not particularly limited as long as it is in a fiber form. Examples of the shape include web, narrow cylinder, cut split yarn, staple, filament, and the like.

The amount of the water-absorbing agent of the present invention added to an absorbent article may vary depending on the type, size, and intended absorption capacities of the absorbent article. When the absorbent article is a disposable diaper or an incontinence pad, the amount of the water-absorbing agent is usually 3 to 20 g per one article;

and when the absorbent article is a sanitary napkin, panty liner, mother's milk pad or the like, the amount is usually 0.2 to 3 g per one article. When the water-absorbing agent is used in a sheet-like article in which the water-absorbing agent is sandwiched between two or more water-absorbing papers or nonwoven fabrics, the suitable amount is usually about 10 to 80 g/$m^2$.

The present invention further is described with reference to the following examples and comparative examples, which do not limit the present invention. The absorption capacities, deodorant effect and antibacterial effect of a deodorant and antibacterial water-absorbing agent alone, and the tests for confirming the effects of an absorbent article using a deodorant and antibacterial water-absorbing agent were measured by the procedures described below. In these examples and comparative examples, "%" means "mass %", and "parts" means "mass parts".

[Procedures of Measurements]

(1) Absorption Capacities

Absorption capacities were measured using an artificial urine having the composition below. The contents of respective components in 100 g of the artificial urine are as follows:

| | |
|---|---|
| Urea | 2.0 g |
| Sodium chloride | 0.8 g |
| Magnesium sulfate hexahydrate | 0.08 g |
| Calcium chloride dihydrate | 0.03 g |
| Ion exchanged water | 97.09 g |

Absorbency under Pressure-free State:

1.0 g of a water-absorbing agent was put in a 250-mesh nylon tea bag, and it was immersed in excess of the artificial urine for 1 hour to absorb it. Then, the tea bag was pulled out and drained liquid off for 15 minutes, and an increase in mass was measured. The increase in mass was determined as the absorbency under pressure-free state.

Absorbency under Load before Shearing:

0.10 g of a water-absorbing agent was put in a cylindrical plastic tube (inner diameter 30 mm, height 60 mm) having a 250-mesh nylon net stretched at the bottom face, and leveled uniformly on the nylon net. Then, a weight (inner diameter 30 mm) was placed on the resin so that it became a load of 60 g/$cm^2$. The plastic tube containing the water-absorbing agent was immersed in an artificial urine filled in a Petri dish (diameter 12 cm) with the nylon net on the lower side and allowed to stand. The increase in the weight of the water-absorbing agent by absorbing the artificial urine was measured after 60 minutes, and ten times the measured value was determined as the absorbency under load before shearing.

Absorbency under Load after Shearing:

A water-absorbing agent adjusted for a particle size on 30 to 100-mesh using a JIS standard sieve was measured accurately for a weight of 3.0 g. On the other hand, 27.0 g of the artificial urine sampled in a 100 ml beaker was kept stirred at 600 rpm using a magnetic stirrer. The total amount of the measured water-absorbing agent was put in the artificial urine stirred in the beaker to form a homogeneous gel. 10.0 g of the formed gel was put in a polyethylene bag with a zipper (85 mm length, 60 mm width) and heat sealed at the inlet. Roller pressing was repeated back and forth 50 times on the bag with a cylindrical roller of 3.5 kg weight, thereby shearing the gel. 1.00 g of the sheared gel was put in a cylindrical plastic tube having a 250-mesh nylon net stretched at the bottom face (the same tube as used in the measurement of the absorbency under load before shearing), and leveled uniformly on the nylon net. Thereafter, the absorbed amount after 60 minutes was measured in the same manner as in the above measurement of the absorbency under load, and ten times the measured value was determined as the absorbency after shearing.

The retention ratio of absorbency under load of sheared gel was calculated according to the following formula:

$$\text{Retention ratio of absorbency under load (\%)} = (\text{Absorbency under load after shearing} / \text{Absorbency under load before shearing}) \times 100$$

(2) Ammonia Deodorization Test

About 0.3 ml of an ammonia reagent (ammonia concentration 26 %) was sampled with a microsyringe and put in a 300 cc flask provided with a glass tube (diameter 5 mm), and the ammonia gas concentration in the flask was adjusted to about 1000 ppm. 1.00 g of a water-absorbing agent was put in the flask and kept at 37 °C, and the ammonia gas concentration was measured with an ammonia gas detector ("KITAGAWA type gas detector" No. 3M, manufactured by GASTEC Corporation) while sucking it from the tip of the glass tube at each of predetermined times (after 5, 10, 30 and 60 minutes).

(3) Test of Antibacterial Property of a Deodorant and Antibacterial Water-absorbing Agent

3.45 g of a sensitive broth medium and 150 ml of ion exchanged water were put in a 300 cc volume flask and dissolved, and then autoclaved. 1.0 g of a test sample was added to the medium and allowed to swell while stirring, and thereafter *Escherichia coli* was inoculated such that the cell number became $1 \times 10^6$ cells/ml.

This sample was shake-cultured at 37 °C, and sampled after 2 hours and 10 hours. The samples were diluted gradually with a sterilized physiological salt solution as needed. The cell number is measured by a mixture plane culturing method. 1 ml of each sample or dilution was put in a sterilized Petri dish, then 20 ml of an agar medium was poured, and dispersed and solidified homogeneously on the dish, and cultured at 37 °C for 2 days.

After culturing, the colony was counted, and the count was multiplied by the diluting ratio to determine the viable cell number. As a blank, the viable cell number when only *Escherichia coli* was inoculated without adding a test sample was $6.1 \times 10^8$ cells/ml after 2 hours, and $8.3 \times 10^9$ cells/ml after 10 hours.

In the same manner, the antibacterial property also was tested for ammonia-producing bacteria. As a blank, the viable cell number when only ammonia-producing bacteria was inoculated without adding a test sample was $8.6 \times 10^8$ cells/ml after 2 hours, and $9.7 \times 10^9$ cells/ml after 10 hours.

(4) Powder Flowability Index

Powder flowability was evaluated by measuring five items of dispersion degree, angle of repose, angle of spatula, homogeneity, and condensation degree, and calculating according to the formula of powder flowability index proposed by Dr. Caar. These indicators of the powder flowability were measured using a powder tester (TYPE "PT-1", manufactured by HOSOKAWA MICRON Corporation).

(5) Hygroscopic Blocking Resistance

5.00 g of a water-absorbing agent was spread uniformly on an aluminum dish to be sampled, and allowed to stand for 3 hours in a thermo-hygrostatic chamber (30 °C, 80 % RH). After the test, the water-absorbing agent on the aluminum dish was sieved using 12-mesh wire gauze, and the amount of the water-absorbing agent remaining on the wire gauze was weighed. A hygroscopic blocking ratio of the tested water-absorbing agent was determined according to the following formula and used as an indicator of the hygroscopic blocking resistance. The smaller the hygroscopic blocking ratio, the better the hygroscopic blocking resistance.

$$\text{Hygroscopic blocking ratio (\%)} = (\text{The amount of the water-absorbing agent remaining on the wire gauze} / \text{Total amount of the water-absorbing agent after the test}) \times 100$$

6) Tests for Confirming the Effects of an Absorbent Article Using a Deodorant and Antibacterial Water-absorbing Agent

Preparation of Model Absorbent Article:

100 g of a water-absorbing agent and 100 g of fluff pulp were mixed by an air-stream type blender, and layered uniformly so that the basis weight became about 400 g/m$^2$. Then, it was pressed at a pressure of 10 kg/cm$^2$ for 30 seconds. The obtained absorption layer was cut into dimensions of 15 cm width and 35 cm length. A tissue paper having the same area as the absorption layer and a polyethylene film having dimensions of 16 cm width and 36 cm length were placed below the absorption layer, and a tissue paper having the same area as the absorption layer and a polypropylene fiber nonwoven fabric having dimensions of 16 cm width and 36 cm length were placed above the absorption layer. Then, the laminate was heat sealed at its peripheries to prepare a model absorbent article.

Test of the Effect for Preventing Offensive Odor:

A liquid in which 0.1 mg of urease was dissolved in 80 ml of fresh urine was added to the center of an absorbent article using a deodorant and antibacterial water-absorbing agent. The absorbent article was put in a 5-liter wide-mouthed bottle and sealed, and it was stored in a thermostat chamber set at 37 °C for 10 hours. Then, the lid of the bottle was lifted in an odorless chamber and smelled, and the intensity of the odor was evaluated by a six-scale described below. The evaluation was carried out by ten panelists confirmed to have the odor-evaluation ability by T&T olfactometry, and their average value was determined.

0: No odor
1: Odor that can be sensed barely (threshold concentration of sense)
2: Weak odor that can be recognized (threshold concentration of recognition)
3: Odor that can be sensed easily
4: Strong odor
5: Very strong odor

Test of Ammonia Odor:

In the above test of the effect for preventing offensive odor, the lid of the wide-mouthed bottle was removed 1 hour and 10 hours after the test sample was allowed to stand in the thermostat chamber set at 37 °C. The concentration of ammonia gas generated in the wide-mouthed bottle was measured by an ammonia gas detector ("KITAGAWA type gas detector" No. 3M, manufactured by GASTEC Corporation).

(7) Performance Tests of Absorbent Article

Absorbed Amount:

A model absorbent article was immersed in a large excess of the artificial urine for 60 minutes, and then placed on a wire gauze. A load of 10 kg was applied uniformly on the absorbent article, and liquid was drained off for 20 minutes. Then, the increase in mass was determined as the absorbed amount.

Rewet Amount:

50 ml of the artificial urine was poured to the center of a model absorbent article. After 10 minutes, ten pieces of filter paper of 10 cm x 10 cm were laminated at the center of the disposable diaper, and a load of 3.5 kg was applied uniformly on the filter paper. After 3 minutes, the increase in mass of the filter paper was measured, which value was determined as the rewet amount. The smaller the rewet amount, the better the surface dryness of the absorbent article.

<u>Example 1</u>

76.6 g of sodium acrylate, 23 g of acrylic acid, 0.5 g of N,N'-methylenebisacrylamide, and 295 g of deionized water were put in a 1-liter capacity reaction vessel made of glass, and the temperature of the content was kept at 5 °C while it was stirred and mixed.

Nitrogen was passed through the content so that the amount of dissolved oxygen was not more than 1 ppm. Then, 1 g of 1 % aqueous solution of hydrogen peroxide, 1.2 g of 0.2 % aqueous solution of ascorbic acid, and 2.4 g of 2 % aqueous solution of 2,2'-azobisamidinopropane dihydrochloride were added to initiate polymerization, which was carried out for about 5 hours. Thus, a hydrous gel polymer (I) having about 25 % concentration of a water-absorbent resin was obtained.

While kneading 100 parts of the hydrous gel polymer (I) by a kneader, 5 parts of 1 % aqueous solution of ethylene glycol diglycidyl ether was added, and kneaded uniformly. Thereafter, 0.17 part of 30% aqueous solution of didecyldimethylammonium gluconate was added and kneaded uniformly. This mixture was dried at 90 °C under reduced pressure, and pulverized by a pin mill. Then, the obtained particles were sieved to adjust the particle size so that the ratio of particles having a size of 150 to 850 µm was about 95 %.

To thus obtained water-absorbing agent, 1.0 part of fine particulate potassium alum ("TAIACE K20" manufactured by DAIMEIKAGAKU KOGYO Corporation; average particle size of 20 µm; pH 4.2 for 1 % aqueous solution) was added and mixed by a Nauta blender for 30 minutes, thereby obtaining a deodorant and antibacterial water-absorbing agent {1}. The results of measured performances of this deodorant and antibacterial water-absorbing agent {1} are shown in Tables 1, 2 and 3.

Example 2

While kneading 100 parts of the hydrous gel polymer (I) obtained in Example 1 by a kneader, 5 parts of 1 % aqueous solution of ethylene glycol diglycidyl ether was added and mixed uniformly. Thereafter, this mixture was dried at about 95 °C under reduced pressure, and pulverized by a pin mill. Then, the obtained particles were sieved to adjust the particle size so that the ratio of particles having a size of 150 to 850 µm was about 95 %.

Next, to 100 parts of this size-adjusted particle product, 2 parts of 5 % concentration solution of ethylene glycol diglycidyl ether (composition of diluting solution: water/methanol = 30/70 mass % mixed solution) was added, and mixed uniformly by a mixer. The obtained product was heated at 130 °C for one hour, and a surface-crosslinked water-absorbent resin powder was obtained.

To this powder, 0.17 part of the same 30 % aqueous solution of didecylthmethylammonium gluconate as used in Example 1 was added, and further 1.0 part of fine particulate potassium alum was added, and mixed uniformly for 30 minutes by a Nauta blender. The obtained product was adjusted for the particle size so that the ratio of particles having a size of 150 to 850 µm was about 95 %, thereby obtaining a deodorant and antibacterial water-absorbing agent {2}. The results of measured performances of this deodorant and antibacterial water-absorbing agent {2} are shown in Tables 1, 2 and 3.

Example 3

According to the same procedure as in Example 1, a hydrous gel polymer (I) having about 25 % concentration of a water-absorbent resin was obtained.

While kneading 100 parts of the hydrous gel polymer (I) by a kneader, 0.75 part of 30 % methanol/water mixed solution (methanol/water ratio = 50/50 mass %) of didecyldimethylammonium carbonate was added, and kneaded uniformly. This mixture was dried at 90 °C under reduced pressure, and pulverized by a pin mill. Then, the obtained particles were sieved to adjust the particle size so that the ratio of particles having a size of 150 to 850 µm was about 95 %.

To thus obtained water-absorbing agent, 1.0 part of fine particulate potassium alum ("TAIACE K20" manufactured by DAIMEIKAGAKU KOGYO Corporation; average particle size of 20 µm; pH 4.2 of 1 % aqueous solution) was added and mixed by a Nauta blender for 30 minutes, thereby obtaining a deodorant and antibacterial water-absorbing agent {3}. The results of measured performances of this deodorant and antibacterial water-absorbing agent {3} are shown in Tables 1, 2 and 3.

Example 4

According to the same procedure as in Example 2 except that the same amount of 30 % aqueous solution of cetyltrimethylammonium gluconate was used in place of 30 % aqueous solution of didecyldimethylammonium gluconate, a deodorant and antibacterial water-absorbing agent {4} was obtained. The results of measured performances of this deodorant and antibacterial water-absorbing agent {4} are shown in Tables 1, 2 and 3.

Example 5

According to the same procedure as in Example 2 except that the same amount of 30 % aqueous solution of didecyldimetbylammonium chloride was used in place of 30 % aqueous solution of didecyldimethylammonium gluconate, a deodorant and antibacterial water-absorbing agent {5} was obtained. The results of measured performances of this deodorant and antibacterial water-absorbing agent {5} are shown in Tables 1, 2 and 3.

Examples 6 and 7

According to the same procedure as in Example 2 except that the added amount of 30 % aqueous solution of didecyldimethylammonium gluconate was changed to 0.7 part (Example 6) or 1.5 parts (Example 7), deodorant and antibacterial water-absorbing agents {6} and {7} were obtained. The results of measured performances of these deodorant and antibacterial water-absorbing agents {6} and {7} are shown in Tables 1, 2 and 3.

Example 8

According to the same procedure as in Example 2 except that the same amount of 30 % aqueous solution of polyhexamethylene biguanidine gluconate was used in place of 30 % aqueous solution of didecyldimethylammonium gluconate, a deodorant and antibacterial water-absorbing agent {8} was obtained. The results of measured performances of this deodorant and antibacterial water-absorbing agent {8} are shown in Tables 1, 2 and 3.

Example 9

According to the same procedure as in Example 2 except that the same amount of 30 % aqueous dispersion of chlorohexidine was used in place of 30 % aqueous solution of didecyldimethylammonium gluconate, a deodorant and antibacterial water-absorbing agent {9} was obtained. The results of measured performances of this deodorant and antibacterial water-absorbing agent {9} are shown in Tables 1, 2 and 3.

Example 10

According to the same procedure as in Example 2 except that the added amount of fine particulate potassium alum was changed to 1.2 parts, a deodorant and antibacterial water-absorbing agent {10} was obtained. The results of measured performances of this deodorant and antibacterial water-absorbing agent {10} are shown in Tables 1, 2 and 3.

Example 11

According to the same procedure as in Example 10 except that 4.0 parts of 30 % aqueous solution of potassium alum was added in place of 1.2 parts of fine particulate potassium alum, a deodorant and antibacterial water-absorbing agent {11} was obtained. The results of measured performances of this deodorant and antibacterial water-absorbing agent {11} are shown in Tables 1, 2 and 3.

Example 12

According to the same procedure as in Example 11 except that the same amount of aluminum sulfate (pH 3.5 of 1 % aqueous solution) was added in place of 4.0 parts of 30 % aqueous solution of potassium alum, a deodorant and antibacterial water-absorbing agent {12} was obtained. The results of measured performances of this deodorant and antibacterial water-absorbing agent {12} are shown in Tables 1, 2 and 3.

Example 13

According to the same procedure as in Example 10 except that the same amount of sodium metaborate was used in place of 1.2 parts of fine particulate potassium alum, a deodorant and antibacterial water-absorbing agent {13} was obtained. The results of measured performances of this deodorant and antibacterial water-absorbing agent {13} are shown in Tables 1, 2 and 3.

Example 14

According to the same procedure as in Example 10 except that the same amount of "FRESHSHIRAIMATSU" (a flavonoid compound manufactured by SHIRAIMATSU PHARMACEUTICAL CO., LTD.) was used in place of 1.2 parts of fine particulate potassium alum, a deodorant and antibacterial water-absorbing agent {14} was obtained. The results of measured performances of this deodorant and antibacterial water-absorbing agent {14} are shown in Tables 1, 2 and 3.

Examples 15 to 25

Using the deodorant and antibacterial water-absorbing agents obtained in Examples 1 to 3, Examples 5 to 8 and Examples 11 to 14, model absorbent articles were prepared.

Absorbent articles using the deodorant and antibacterial water-absorbing agent {1} of Example 1, the deodorant and antibacterial water-absorbing agent {2} of Example 2, the deodorant and antibacterial water-absorbing agent {3} of Example 3, the deodorant and antibacterial water-absorbing agent {5} of Example 5, the deodorant and antibacterial water-absorbing agent {6} of Example 6, the deodorant and antibacterial water-absorbing agent {7} of

Example 7, the deodorant and antibacterial water-absorbing agent {8} of Example 8, the deodorant and antibacterial water-absorbing agent {11} of Example 11, the deodorant and antibacterial water-absorbing agent {12} of Example 12, the deodorant and antibacterial water-absorbing agent {13} of Example 13 and the deodorant and antibacterial water-absorbing agent {14} of Example 14 are determined as (a), (b), (c), (d), (e), (f), (g), (h), (i), (j) and (k), respectively. The results of measured performances of these absorbent articles are shown in Table 4.

Comparative Example 1

The hydrous gel polymer (I) obtained in Example 1 was dried at 90 °C under reduced pressure, and pulverized by a pin mill. Then, the obtained particles were adjusted for the particle size so that the ratio of particles having a size of 150 to 850 μm was about 95 %, thereby obtaining a comparative water-absorbing agent [1]. The results of measured performances of this water-absorbing agent [1] are shown in Tables 1, 2 and 3.

Comparative Example 2

According to the same procedure as in Example 2 except that fine particulate potassium alum was not added, a comparative antibacterial water-absorbing agent [2] was obtained. The results of measured performances of this water-absorbing agent [2] are shown in Tables 1, 2 and 3.

Comparative Example 3

According to the same procedure as in Example 2 except that 30 % aqueous solution of didecyldimethylammonium gluconate was not added, a comparative water-absorbing agent [3] was obtained. The results of measured performances of this water-absorbing agent [3] are shown in Tables 1, 2 and 3.

Comparative Example 4

According to the same procedure as in Example 3 except that fine particulate potassium alum was not used, a comparative antibacterial water-absorbing agent [4] was obtained. The results of measured performances of this comparative antibacterial water-absorbing agent [4] are shown in Tables 1, 2 and 3.

Comparative Example 5

According to the same procedure as in Example 8 except that fine particulate potassium alum was not used, a comparative antibacterial water-absorbing agent [5] was obtained. The results of measured performances of this comparative antibacterial water-absorbing agent [5] are shown in Tables 1, 2 and 3.

Comparative Example 6

According to the same procedure as in Example 9 except that fine particulate potassium alum was not used, a comparative antibacterial water-absorbing agent [6] was obtained. The results of measured performances of this comparative antibacterial water-absorbing agent [6] are shown in Tables 1, 2 and 3.

Comparative Example 7

According to the same procedure as in Example 11 except that 30 % aqueous solution of didecyldimethylammonium gluconate was not used, a comparative water-absorbing agent [7] was obtained. The results of measured performances of this comparative water-absorbing agent [7] are shown in Tables 1, 2 and 3.

Comparative Example 8

According to the same procedure as in Example 12 except that 30 % aqueous solution of didecyldimethylammonium gluconate was not used, a comparative water-absorbing agent [8] was obtained. The results of measured performances of this comparative water-absorbing agent [8] are shown in Tables 1, 2 and 3.

Comparative Example 9

According to the same procedure as in Example 13 except that 30 % aqueous solution of didecyldimethylammonium gluconate was not used, a comparative water-absorbing agent [9] was obtained. The results of measured performances of this comparative water-absorbing agent [9] are shown in Tables 1, 2 and 3.

Comparative Example 10

According to the same procedure as in Example 14 except that 30 % aqueous solution of didecyldimethylammonium gluconate was not used, a comparative water-absorbing agent [10] was obtained. The results of measured performances of this comparative water-absorbing agent [10] are shown in Tables 1, 2 and 3.

Comparative Examples 11 to 20

In the same manner as described in Examples 15 to 25, using the water-absorbing agent [1] of Comparative Example 1, the antibacterial water-absorbing agent [2] of Comparative Example 2, the water-absorbing agent [3] of Comparative Example 3, the antibacterial water-absorbing agents [4], [5] and [6] of Comparative Examples 4 to 6, and the water-absorbing agents [7], [8], [9] and [10] of Comparative Examples 7 to 10, comparative model absorbent articles (l), (m), (n), (o), (p), (q), (r), (s), (t) and (u) were obtained. The results of measured performances of these comparative absorbent articles are shown in Table 4.

Table 1

Results of Tests for Absorption Capacities, Powder Flowability and Hygroscopic Blocking Resistance

| | | Absorbency under pressure-free state (g/g) | Absorbency under load before shearing (g/g) | Absorbency under load after shearing (g/g) | Retention Ratio of Absorbency Under load (%) | Powder flowability index | Hygroscopic blocking ratio (%) |
|---|---|---|---|---|---|---|---|
| Example | 1 | 54 | 18 | 13 | 72 | 79 | 65 |
| | 2 | 48 | 24 | 20 | 83 | 80 | 45 |
| | 3 | 52 | 17 | 12 | 70 | 78 | 68 |
| | 4 | 48 | 24 | 20 | 83 | 78 | 45 |
| | 5 | 47 | 24 | 19 | 79 | 78 | 48 |
| | 6 | 47 | 23 | 20 | 87 | 79 | 55 |
| | 7 | 46 | 21 | 20 | 95 | 78 | 60 |
| | 8 | 48 | 22 | 18 | 82 | 77 | 48 |
| | 9 | 47 | 23 | 19 | 83 | 79 | 46 |
| | 10 | 45 | 20 | 18 | 90 | 81 | 41 |
| | 11 | 46 | 22 | 18 | 82 | 80 | 40 |
| | 12 | 44 | 21 | 19 | 90 | 78 | 35 |
| | 13 | 43 | 21 | 16 | 76 | 77 | 50 |
| | 14 | 45 | 21 | 17 | 81 | 77 | 52 |
| Comparative Example | 1 | 57 | 12 | 7 | 58 | 75 | 86 |
| | 2 | 51 | 22 | 18 | 82 | 73 | 65 |
| | 3 | 49 | 23 | 21 | 91 | 80 | 50 |
| | 4 | 51 | 22 | 18 | 82 | 72 | 72 |
| | 5 | 51 | 20 | 16 | 80 | 72 | 74 |
| | 6 | 50 | 22 | 17 | 77 | 74 | 73 |
| | 7 | 48 | 19 | 15 | 79 | 75 | 75 |
| | 8 | 46 | 18 | 13 | 72 | 73 | 70 |
| | 9 | 49 | 20 | 17 | 85 | 72 | 73 |
| | 10 | 48 | 18 | 14 | 78 | 72 | 77 |

Table 2

Results of Tests for Antibacterial Properties against *Escherichia coli* and Ammonia-producing Bacteria

| | | *Escherichia coli* (cell number/ml) | | Ammonia-producing bacteria (cell number/ml) | |
|---|---|---|---|---|---|
| | | After 2 hours | After 10 hours | After 2 hours | After 10 hours |
| Example | 1 | 50 | 10 | $3.5 \times 10^2$ | $3.8 \times 10^2$ |
| | 2 | $1.8 \times 10^2$ | 5 | $7.8 \times 10^2$ | $8.8 \times 10^2$ |
| | 3 | $5.0 \times 10^2$ | 35 | $6.0 \times 10^2$ | $7.5 \times 10^2$ |
| | 4 | $1.5 \times 10^2$ | 20 | 5 | 0 |
| | 5 | $4.8 \times 10^2$ | 80 | $1.2 \times 10^3$ | $2.2 \times 10^3$ |
| | 6 | 0 | 0 | 10 | 5 |
| | 7 | 0 | 0 | 0 | 0 |
| | 8 | $8.5 \times 10^2$ | $2.1 \times 10^2$ | $4.3 \times 10^3$ | $1.5 \times 10^3$ |
| | 9 | $6.2 \times 10^2$ | $1.7 \times 10^2$ | $7.8 \times 10^2$ | 96 |
| | 10 | 15 | 0 | 85 | 75 |
| | 11 | 20 | 0 | $2.1 \times 10^2$ | $1.3 \times 10^2$ |
| | 12 | 25 | 0 | 0 | 0 |
| | 13 | $5.5 \times 10^2$ | $3.3 \times 10^2$ | $5.6 \times 10^2$ | $2.7 \times 10^3$ |
| | 14 | $7.4 \times 10^2$ | $1.8 \times 10^2$ | $8.4 \times 10^2$ | $5.6 \times 10^2$ |
| Comparative Example | 1 | $4.1 \times 10^8$ | $4.8 \times 10^9$ | $6.5 \times 10^8$ | $8.1 \times 10^9$ |
| | 2 | $2.2 \times 10^2$ | 10 | $9.4 \times 10^3$ | $1.1 \times 10^3$ |
| | 3 | $3.5 \times 10^8$ | $4.2 \times 10^9$ | $5.5 \times 10^8$ | $7.0 \times 10^9$ |
| | 4 | $6.0 \times 10^2$ | $4.5 \times 10^2$ | $6.8 \times 10^3$ | $2.5 \times 10^4$ |
| | 5 | $9.3 \times 10^2$ | $3.3 \times 10^2$ | $8.8 \times 10^3$ | $4.7 \times 10^3$ |
| | 6 | $7.7 \times 10^2$ | $2.7 \times 10^2$ | $9.2 \times 10^2$ | $1.8 \times 10^2$ |
| | 7 | $5.2 \times 10^7$ | $1.6 \times 10^9$ | $2.2 \times 10^8$ | $5.0 \times 10^9$ |
| | 8 | $4.8 \times 10^8$ | $6.2 \times 10^9$ | $7.9 \times 10^8$ | $8.3 \times 10^9$ |
| | 9 | $2.6 \times 10^8$ | $1.8 \times 10^9$ | $4.7 \times 10^8$ | $8.1 \times 10^9$ |
| | 10 | $1.4 \times 10^9$ | $9.1 \times 10^9$ | $1.8 \times 10^9$ | $9.6 \times 10^9$ |

Table 3

Results of Ammonia Deodorization Test

| | | Ammonia Concentration (ppm) | | | |
|---|---|---|---|---|---|
| | | After 5 minutes | After 10 minutes | After 30 minutes | After 60 minutes |
| Example | 1 | 480 | 200 | 100 | 80 |
| | 2 | 455 | 160 | 125 | 85 |
| | 3 | 480 | 190 | 110 | 85 |
| | 4 | 605 | 180 | 130 | 75 |
| | 5 | 600 | 230 | 210 | 140 |
| | 6 | 550 | 150 | 130 | 80 |
| | 7 | 280 | 140 | 95 | 70 |
| | 8 | 560 | 210 | 200 | 155 |
| | 9 | 510 | 190 | 160 | 110 |
| | 10 | 180 | 95 | 55 | 50 |
| | 11 | 350 | 170 | 150 | 105 |
| | 12 | 100 | 80 | 50 | 35 |
| | 13 | 430 | 220 | 185 | 155 |
| | 14 | 505 | 205 | 150 | 95 |
| Comparative Example | 1 | 1000 | 1000 | 1000 | 1000 |
| | 2 | 1000 | 1000 | 940 | 770 |
| | 3 | 650 | 620 | 505 | 380 |
| | 4 | 1000 | 1000 | 950 | 800 |
| | 5 | 1000 | 860 | 645 | 685 |
| | 6 | 960 | 650 | 510 | 410 |
| | 7 | 525 | 305 | 340 | 455 |
| | 8 | 705 | 430 | 480 | 605 |
| | 9 | 680 | 355 | 370 | 540 |
| | 10 | 655 | 330 | 405 | 600 |

Table 4

Results of Performance Tests for Absorbent Article

| | | Absorbed amount (g/article) | Rewet amount (g) | Effect for preventing offensive odor | Ammonia gas concentration (ppm) | |
|---|---|---|---|---|---|---|
| | | | | | After 1 hour | After 10 hour |
| Example | a | 495 | 0.2 | 1.0 | 20 | 320 |
| | b | 460 | 0.1 | 1.0 | 20 | 350 |
| | c | 460 | 0.2 | 1.0 | 22 | 360 |
| | d | 455 | 0.2 | 0.8 | 18 | 315 |
| | e | 455 | 0.2 | 1.5 | 29 | 370 |
| | f | 460 | 0.2 | 1.0 | 20 | 280 |
| | g | 460 | 0.2 | 1.4 | 34 | 377 |
| | h | 455 | 0.1 | 1.1 | 30 | 292 |
| | i | 455 | 0.1 | 0.7 | 8 | 75 |
| | j | 445 | 0.2 | 1.2 | 18 | 175 |
| | k | 450 | 0.3 | 0.9 | 15 | 124 |
| Comparative Example | l | 485 | 1.1 | 4.7 | 250 | 750 |
| | m | 490 | 0.5 | 3.5 | 240 | 650 |
| | n | 445 | 0.2 | 4.4 | 150 | 770 |
| | o | 460 | 0.3 | 3.8 | 235 | 784 |
| | p | 465 | 0.2 | 3.6 | 206 | 722 |
| | q | 460 | 0.6 | 3.9 | 188 | 747 |
| | r | 455 | 0.3 | 4.2 | 105 | 705 |
| | s | 450 | 0.7 | 4.5 | 173 | 758 |
| | t | 455 | 0.2 | 4.3 | 147 | 712 |
| | u | 455 | 0.3 | 4.4 | 215 | 765 |

The deodorant and antibacterial water-absorbing agent of the present invention has the following characteristics and effects.

(1) Compared to water-absorbing agents in which only an antibacterial component is contained or supported, the water-absorbing agent of the present invention is excellent in the deodorant property in a relatively short time at the

initial period of absorption, and maintains the deodorant property for a long time.

(2) Compared to conventional water-absorbing agents, because the water-absorbing agent of the present invention can inhibit decomposition and putrefaction by fungi, microorganism, bacteria, etc. of organic substances contained in the absorbed urine, blood, body fluids and the like, generation of offensive odor is small even when wearing an absorbent article in which urine or the like is absorbed for a long time.

(3) Because the water-absorbing agent of the present invention can inhibit decomposition and putrefaction of water-absorbed gel by fungi, microorganism, bacteria and the like, it can maintain excellent absorption capacities over a long time. Thus, when the water-absorbing agent of the present invention is used in absorbent articles such as disposable diapers, sanitary napkins and the like, it is excellent in repetitive absorptivity, and maintains excellent dry feeling over a long time.

(4) Because generation of alkali substances such as ammonia is inhibited, and there is a deodorant agent on the surface of the water-absorbing agent having neutralization and adsorption functions, the pH of the absorbed urine or the like does not become alkaline, so that it is safe for the skin.

(5) Because the water-absorbing agent of the present invention is excellent in powder flowability as well as in hygroscopic blocking resistance, it has excellent powder handling properties, and can be used in absorbent articles in the same manner as conventional powdery water-absorbent resins.

(6) By using the deodorant and antibacterial water-absorbing agent of the present invention in absorbent articles such as disposable diapers, sanitary napkins and the like, both deodorant and antibacterial functions can be provided to the absorbent articles, and also generation of an offensive odor is inhibited, and irritation on the skin and generation of rash are reduced. In addition, absorption capacities are improved.

(7) Because growth of fungi, microorganisms, bacteria, etc. is inhibited, the water-absorbing agent of the present invention is safe in the operation for exchanging or disposing absorbent articles after use, so that the risk of a secondary infection for an operator is low.

INDUSTRIAL APPLICABILITY

Because of the above-mentioned effects, the deodorant and antibacterial water-absorbing agent of the present invention is particularly useful in various types of absorbent articles, such as disposable diapers for adults and infants, incontinence pads, sanitary napkins, panty liners, mother's milk pads, parturition mats, underpads for medical treatment, and the like.

Furthermore, the water-absorbing agent of the present invention is useful for gelling agents and excretion treating agents for various types of body fluids that generate an offensive odor by putrefaction, such as urine of pets, waste blood and the like. Also, it is useful when producing sheet-like or tape-like absorbent articles, such as pet sheets, drip absorbing materials and the like. In addition, it is also useful in applications utilizing gel of a water-absorbent resin that has absorbed water (e.g. cold insulators, artificial snow, water beds, etc.), and applications accompanied by generation of an offensive odor by putrefaction, such as sludge solidifying agents, dew-condensation preventing wall materials, and the like.

**Claims**

**1.** A deodorant and antibacterial water-absorbing agent comprising:

(A) a water-absorbent resin;
(B) a compound having an antibacterial function with respect to ammonia-producing bacteria; and
(C) an agent having a neutralization ability or neutralization and adsorption abilities with respect to ammonia;
wherein (A) and (B) are mixed and/or bonded.

**2.** The water-absorbing agent according to claim 1, wherein (A) is a water-absorbent resin that is water-insoluble and has acrylic acid and salts thereof as main constituent units, and the acrylic acid units are from 20 to 50 mol % with respect to a total of the acrylic acid units and the acrylic acid salt units.

**3.** The water-absorbing agent according to claim 1, wherein (B) is at least one selected from the group consisting of a quaternary ammonium salt compound (B1) having at least one $C_6$ to $C_{30}$ alkyl group, a polymethylene biguanidine compound (B2), and a chlorohexidine compound (B3).

**4.** The water-absorbing agent according to claim 3, wherein (B1) has a quaternary ammonium group having at least one $C_8$ to $C_{20}$ alkyl group as a cation component, and has at least one carboxylic acid anion selected from aliphatic monocarboxylic acids, aliphatic oxycarboxylic acids, and aliphatic polycarboxylic acids as an anion component.

5. The water-absorbing agent according to claim 1, wherein the agent (C) having a neutralization ability or neutralization and adsorption abilities with respect to ammonia is at least one selected from the group consisting of an inorganic acid salt (C1), a flavonoid compound (C2), and cyclodextrin (C3).

6. The water-absorbing agent according to claim 5, wherein (C1) is an inorganic salt of an acid selected from strong acids and carbonic acid.

7. The water-absorbing agent according to claim 5, wherein (C1) is a salt of an inorganic strong acid and a weak base and/or an inorganic hydrogencarbonate, and a pH of 1 mass % aqueous solution of (C1) is from 3.0 to 6.5.

8. The water-absorbing agent according to claim 1, wherein a ratio of (A) : (B) : (C) is 100 : (0.01 to 5) : (0.05 to 10) on mass basis.

9. The water-absorbing agent according to claim 3, comprising: a water-absorbing agent powder in which (B1) is built in (A) or supported by (A); and an inorganic salt of an acid selected from strong acids and carbonic acid.

10. The water-absorbing agent according to claim 3, wherein (A) has acid radicals at side chains, at least a part of the acid radicals existing in a form of a quaternary ammonium salt bonded to (B1), and a content of quaternary nitrogen atoms in (A) is from $2 \times 10^{-4}$ to 0.8 mass %.

11. The water-absorbing agent according to claim 1, wherein with respect to an artificial urine: an absorbency under pressure-free state is at least 40 g/g; an absorbency under load (60 $g/cm^2$) is at least 15 g/g; an absorbency under load (60 $g/cm^2$) of sheared gel is at least 10 g/g; and a retention ratio of absorbency under load is from 60 to 100 %.

12. The water-absorbing agent according to claim 1, wherein in an ammonia deodorization test, an ammonia gas concentration after allowing the water-absorbing agent to stand at 37 °C for 10 minutes is not more than 50 % of an initial ammonia gas concentration.

13. The water-absorbing agent according to claim 1 having an antibacterial effect in which a viable cell number measured by a mixture plane culturing method after inoculating ammonia-producing bacteria in the water-absorbing agent and culturing them for 2 hours is not more than one ten-thousandth of an initial viable cell number at the time of inoculation.

14. A process for producing the deodorant and antibacterial water-absorbing agent according to claim 1, comprising: mixing and kneading (B) with a hydrous gel of (A); drying and pulverizing the mixture; and then mixing it with (C) in a form of an aqueous solution or fine powder.

15. A process for producing the deodorant and antibacterial water-absorbing agent according to claim 1, comprising: mixing a powder of (A) and an aqueous solution of (B); and then mixing the mixture with (C) in a form of an aqueous solution, aqueous dispersion or fine powder.

16. An absorbent article which has an absorption layer in which the deodorant and antibacterial water-absorbing agent according to any of claims 1 to 13 or a deodorant and antibacterial water-absorbing agent obtainable by the process according to claim 14 or 15 is held by a support comprising a fibrous material (D).

# INTERNATIONAL SEARCH REPORT

International application No.
PCT/JP98/03725

A. CLASSIFICATION OF SUBJECT MATTER
Int.Cl[6] B01J20/26, A61F5/44, A61F13/15

According to International Patent Classification (IPC) or to both national classification and IPC

B. FIELDS SEARCHED

Minimum documentation searched (classification system followed by classification symbols)
Int.Cl[6] B01J20/26, A61F5/44, A61F13/15

Documentation searched other than minimum documentation to the extent that such documents are included in the fields searched
Jitsuyo Shinan Koho 1926-1996 Toroku Jitsuyo Shinan Koho 1994-1998
Kokai Jitsuyo Shinan Koho 1971-1997 Jitsuyo Shinan Keisai Koho 1996-1998

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP, 7-507825, A (Courtaulds Fibers (Holdings) Ltd.),<br>31 August, 1995 (31. 08. 95),<br>Claims 1 to 5 ; page 6, upper left column, lines 2 to 17<br>& WO, 9325735, A & EP, 644958, A | 1, 8<br>2-7, 9-16 |
| Y | JP, 9-248454, A (Sanyo Chemical Industries, Ltd.), 22 September, 1997 (22. 09. 97), Claims 1 to 10 ; page 5, right column, line 23 to page 6, left column, line 9 ; Tables 1 to 3 (Family: none) | 2-7, 9-16 |
| Y | JP, 7-39749, A (Esupo K.K.), 10 February, 1995 (10. 02. 95), Claim 1 ; Examples 2, 10 (Family: none) | 5-7, 9 |
| Y | JP, 5-269164, A (Kyowa Hakko Kogyo Co., Ltd.), 19 October, 1993 (19. 10. 93), Claim 1 ; page 2, left column, lines 47, 48 (Family: none) | 5 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 September, 1998 (24. 09. 98) | 6 October, 1998 (06. 10. 98) |
| Name and mailing address of the ISA/<br>Japanese Patent Office | Authorized officer |
| Facsimile No. | Telephone No. |

Form PCT/ISA/210 (second sheet) (July 1992)